# EUROPÄISCHE PATENTSCHRIFT

(11) **EP 2 324 865 B1**
(45) Veröffentlichungstag und Bekanntmachung des Hinweises auf die Patenterteilung: **07.01.2015**
(21) Anmeldenummer: 10175171.7
(22) Anmeldetag: 03.09.2010
(51) Int. Cl.: A61L 31/02, A61L 31/14, A61L 31/08

(54) **Implantat**
Implant
Implant

(30) Priorität: 28.09.2009 US 246173 P
(43) Veröffentlichungstag der Anmeldung: 25.05.2011
(73) Patentinhaber: Biotronik VI Patent AG, 6341 Baar (CH)
(72) Erfinder: Bayer, Ullrich, 18211 Admannshagen-Bargeshagen (DE); Gerold, Bodo, 91225 Zellingen (DE)
(74) Vertreter: Lindner-Vogt, Karin L.

(56) Entgegenhaltungen:
- US-A1- 2007 156 231
- US-A1- 2007 250 155
- US-A1- 2007 282 432
- US-A1- 2008 312 736
- US-A1- 2009 187 258
- US-A1- 2009 198 320

## Beschreibung

Die vorliegende Erfindung betrifft ein ein Implantat, insbesondere einer intraluminalen Endoprothese, mit einem Körper enthaltend metallisches Material, enthaltend eine Eisenlegierung.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, insbesondere Wire-Mesh-Stents, Befestigungselemente für Knochen, beispielsweise Schrauben, Platten oder Nägel, Marknägel, Spiralbündelnägel, Kirschnerdrähte, Drähte für Septumokkluder, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heute werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatz zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff zumindest teilweise ein Metall enthält, vorzugsweise Eisen, Mangan, Zink und/oder Wolfram, insbesondere eine Eisenbasislegierung (im Folgenden kurz: Eisenlegierung).

Bei der Realisierung biodegradierbarer Implantate wird angestrebt, die Degradierbarkeit entsprechend der angestrebten Therapie bzw. der Anwendung des jeweiligen Implantats (koronar, intracranial, renal etc.) zu steuern. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen erleidet. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, die Durchlässigkeit des Gefäßes zu gewährleisten, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d. h. erst nach einer Verweildauer in dem behandelten Organismus von ca. 2 Jahren. Dies bedeutet, dass sich der Kollapsdruck bei eisenhaltigen Implantaten für diese Anwendung über die Zeit zu langsam verringert.

Eine lange Verweildauer von Implantaten kann auch dann zu Komplikationen führen, wenn das in Auflösung befindliche Implantat aufgrund seiner ferromagnetischen Eigenschaften die Untersuchung des behandelten Patienten im Magnetresonanztomograph nicht erlaubt. Weiterhin kann bei orthopädischen Implantaten (z. B. bei Knochenplatten) die Bildung von neuer Knochensubstanz zu mechanischen Spannungen zwischen dem zu langsam degradierenden Implantat und der Knochensubstanz führen. Dies erzeugt insbesondere bei Kindern und Heranwachsenden Knochendeformationen bzw. -fehlbildungen. Für derartige Anwendungen ist es daher wünschenswert, wenn insbesondere das Einsatzpotential von Eisenbasis-Implantaten durch schnellere Degradation erweitert werden könnte.

Im Stand der Technik sind bereits unterschiedliche Mechanismen der Degradationskontrolle von Implantaten beschrieben worden. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z. B. lokale Querschnittsveränderungen) der Stent-Oberfläche (z. B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende bzw. eine zu große Variabilität der Degradation des Implantats.

Aus den Druckschriften EP 0 923 389 B1 bzw. WO 99/03515 A2 oder WO 2007/124230 A1 sind Implantate bekannt, welche in vivo durch Korrosion degradierbar sind. Das Material der bekannten Implantate enthält Eisen als Hauptbestandteil sowie in einer bestimmten, vorgegebenen Konzentration Kohlenstoff. Der Nachteil dieser Legierungen besteht darin, dass das reine Zweistoffsystem aus Kohlenstoff und Eisen mit zunehmendem Kohlenstoffgehalt sehr stark an Duktilität verliert, ohne dass in gleichem Maße die Korrosionsbeständigkeit sinkt.

In der EP 08172105.2 werden verschiedenen Eisenlegierungen, beispielsweise ein auf Fe und Mn basierendes Legierungssystem, beschrieben, die zwar gegenüber Reineisen eine leicht erhöhte Korrosionsanfälligkeit in einem den physiologischen Bedingungen nachempfundenen künstlichen Medium zeigen, jedoch immer noch zu langsam degradieren. Nachteilig ist ferner, dass Mn die kubisch flächenzentrierte austenitische Phase des Eisens stabilisiert. Dadurch werden in Abhängigkeit des Gehaltes an weiteren Legierungselementen und der vorangegangenen Wärmebehandlung sowie des konkreten Mn- Gehaltes selbst, Mischgefüge aus austenitischen und kubisch raumzentrierten ferritischen Phasen erzeugt. Der jeweilige prozentuale Anteil dieser Phasen kann auf Grund dieser zahlreichen Einflussgrößen chargenabhängig stark schwanken. Das daraus resultierende unterschiedliche Verfestigungsverhalten von geschnittenen Stents macht eine Dimensionierung, welche auf der Annahme von bestimmten Festigkeits- und Dehnungswerten beruht, schwierig bzw. unmöglich.

Die US 2007/0156231 A1 offenbart ein Implantat mit einer veränderten oberflächennahen Grenzschicht. Das Implantat wird hergestellt durch ein Plasmaimplantationsverfahren in einer Vakuum-Kammer. Hier werden Ionen in das metallische Material implantiert, in dem sie beschleunigt und auf einen so genannten Pre-Stent geschossen werden. Die Beschleunigung der Degradation des Stentmaterials wird jedoch hier nicht behandelt.

Folglich besteht die Aufgabe der vorliegenden Erfindung darin, ein Implantat anzugeben, das eine Degradation des Implantats im gewünschten. Zielkorridor aufweist, insbesondere bei Implantaten mit einer Eisenbasislegierung in einem kürzeren Zeitraum, ohne dass ein Duktilitätsverlust auftritt. Dabei soll die Degradation zu einem kontrollierbaren Zeitpunkt stattfinden und auch kompliziert geformte Implantate mit den gewünschten Degradationseigenschaften ausgestattet werden können. Entsprechend besteht die Aufgabe der Erfindung außerdem darin, ein derartiges Implantat zu schaffen.

Ein Verfahren umfassend die folgenden Schritte:
a) Bereitstellen eines ersten Teils des Implantatkörpers,
b) Durchführung einer Wärmebehandlung, welche den Kohlenstoff-Gehalt und/oder den Bor-Gehalt und den Stickstoff-Gehalt in dem Gefüge einer oberflächenahen Grenzschicht in dem ersten Teil des Implantatkörpers derart verändert, dass eine Verspannung des Gitters oder eine Gitterumwandlung, ggf. nach einer nachfolgenden mechanischen Beanspruchung, in der oberflächennahen Grenzschicht erzielt wird.

Bei der vorliegenden Erfindung umfasst der Körper des Implantats mindestens einen Teil des Implantats, vorzugsweise den Hauptteil des Implantats, welcher die mechanische Integrität des Implantats bewirkt. Der erste Teil des Implantatkörpers kann dabei einen Teil dieses Hauptteils des Implantats oder den gesamten Hauptteil des Implantats umfassen. Die obige Formulierung schließt demnach einen einstückigen Implantatkörper nicht aus. Diese Variante ist durch den Sonderfall der vorliegenden Erfindung, dass lediglich ein einziger erster Teil des Implantatkörpers existiert, ausdrücklich umfasst.

Unter dem Begriff "Gefüge" wird im Folgenden die Anordnung der Bestandteile von Feststoffen (Festkörpern), insbesondere die Anordnung der Kristallite (Körner), Poren, amorphen Bereiche und Korngrenzbereiche des Implantatkörpers verstanden. Ferner wird unter dem Ausdruck "oberflächennahes Gefüge" der Volumenbereich des Gefüges des entsprechenden Teils des Implantatkörpers verstanden, welcher sich von der Oberfläche bis in eine bestimmte (geringe) Tiefe des Implantatkörpers erstreckt. Dieser sich von der Oberfläche bis in eine bestimmte (geringe) Tiefe erstreckende Volumenbereich wird auch als "oberflächennah angeordnete Grenzschicht" oder kurz als "Grenzschicht" bezeichnet.

Der Vorteil des Verfahrens besteht darin, dass mittels eines sehr einfachen und kostengünstigen Verfahrens die Gefügeeigenschaften in einer Grenzschicht, d. h. in einem oberflächennahen Bereich, derart geändert werden, dass sich bei weiterer mechanischer Belastung Risse in der oberflächennahen Grenzschicht bilden, welche die Degradation in dem behandelten Teil des Implantatkörpers beschleunigen. Der durch das Verfahren verwirklichte Degradationszeitraum liegt im Bereich von 3 bis 9 Monaten. Insbesondere kann die Degradationszeit gegenüber der Degradationszeit eines ohne Wärmebehandlung hergestellten Implantats auf höchstens 75%, vorzugsweise höchstens 60% verringert werden.

Ein weiterer Vorteil der erfindungsgemäßen Lösung besteht darin, dass durch die Wahl des Substratwerkstoffs optimale mechanische Eigenschaften des Implantats, insbesondere eine ausreichende Duktilität, in dem nicht zur Grenzschicht gehörenden Volumenanteil des Implantats beibehalten werden. Es wird lediglich ein nahe an der Oberfläche liegender (sehr geringer) Volumenanteil erzeugt, welcher zum Grundmaterial abweichende Degradationseigenschaften aufweist. Diese ergeben sich aus den durch Gitterverspannung oder der geänderten Gitterstruktur in der Grenzschicht durch die aufgrund der Änderung der Zusammensetzung bewirkten Eigenspannungen in der Grenzschicht. Durch eine mechanische Belastung, insbesondere bei einer Dilatation des Implantats, wird ggf. die Änderung der Gitterstruktur induziert und führt zu Rissen. Auch bei mechanischer Belastung von Bereichen mit Gitterverspannungen erzeugen diese Eigenspannungen Risse. Die Erfindung verwirklicht somit eine besonders vorteilhafte Kombination von Volumen- und Grenzbereichseigenschaften. Im Grenzbereich wird außerdem ein Gradient der entsprechenden veränderten Zusammensetzung erzeugt.

Es wird nach dem Schritt b) ein zweiter Teil des Implantatkörpers bereitgestellt, danach der zweite Teil des Implantatkörpers so über dem ersten Teil angeordnet, dass der zweite Teil zumindest in einem Teilbereich der oberflächennahen Grenzschicht an den ersten Teil des Implantatkörpers angrenzt und anschließend das System aus erstem und zweitem Teil des Implantatkörpers einer Wärmebehandlung gemäß Schritt b) unterzogen.

In diesem Ausführungsbeispiel wird ein Mehrlagenverbundwerkstoff erzeugt, bei dem der erste Teil und der zweite Teil des Implantatkörpers Bereiche ausbilden, die in ihrer Zusammensetzung, insbesondere hinsichtlich des Kohlenstoff-, des Bor- und des Stickstoffgehalts, unterschiedlich sind. Über den Querschnitt des Implantatkörpers wechseln Bereiche mit der Ausgangszusammensetzung des Implantatkörpers und (Grenz-)Bereiche mit einer hinsichtlich des Kohlenstoff-, des Bor- und des Stickstoffgehalts veränderter Zusammensetzung ab. Die Wärmebehandlung nach der Anordnung des zweiten Teils bewirkt, dass die durch die Wärmebehandlung des ersten Teils ausgebildete Grenzschicht eine Verbindungsschicht (Interface) zwischen dem ersten und dem zweiten Teil bildet und durch Diffusionsprozesse das zweite Teil an dem ersten Teil des Implantatkörpers festlegt. Eine derartige Sandwichstruktur weist eine vorteilhafte Kombination von Degradationseigenschaften und mechanischen Eigenschaften auf.

Es soll an dieser Stelle darauf hingewiesen werden, dass die Ausbildung einer oben dargestellten Sandwichstruktur für die Wirksamkeit der vorliegenden Erfindung nicht notwendig ist. Die oben dargestellte Ausführungsform stellt lediglich eine - vorteilhafte - Variante der Erfindung dar. Die Erfindung kann einerseits dadurch verwirklicht werden, dass der Implantatkörper lediglich aus einem (einzigen) ersten Teil besteht, und andererseits dadurch realisiert werden, dass der Implantatkörpers aus einem ersten Teil und einer Mehrzahl von zweiten Teilen besteht, wobei der Schritt b) in vorteilhafter Weise nach jeder Anordnung eines weiteren zweiten Teils über den darunter liegenden Teilen des Implantatkörpers wiederholt wird. Somit ist vorstellbar, dass bspw. über einen Querschnitt des Implantats von 100 µm zwischen 5 und 20 Verbundlagen angeordnet werden können.

Nachfolgend werden einige Möglichkeiten dargestellt, wie durch eine Wärmebehandlung eine Gitterverspannung oder eine Gitterumwandlung in der oberflächennahen Grenzschicht erzielt werden kann. Grundsätzlich kann durch eine Wärmebehandlung
I. die Anreicherung von Fe-Verbindungen in der Grenzschicht und eine hierdurch verursachte Gitterverspannung oder
II. die in der Ausgangszusammensetzung stabilisierte z. B. austenitische Phase derart destabilisiert werden, dass sie, beispielsweise durch eine nachfolgende mechanische Belastung, in das härtere und rissanfälligere z. B. martensitische Gitter umklappt.

Die Variante I wird durch die in den nachfolgenden Abschnitten 1. bis 3. dargestellten Lösungen realisiert. Diese Variante hat den zusätzlichen Vorteil, dass die Fe-Verbindungen Lokalelemente bilden können, welche die Degradation zusätzlich beschleunigen.

Die Variante II wird durch die in Abschnitt 4. dargestellte Lösung realisiert. Bei dieser Variante wird das austenitische Gitter durch die Wärmebehandlung metastabil und klappt in die martensitische Gefügeform um.

Welche der beiden Varianten I oder II angewendet werden kann, hängt davon ab, welche Ausgangszusammensetzung der Implantatkörper aufweist. Variante I wird für Fe-Verbindungen, in denen die austenitische Phase nicht stabilisiert ist (z. B. bei einem geringen Mn-Anteil und/oder einem geringen C-Anteil), angewendet. Variante II kommt für Ausgangszusammensetzungen in Frage, in denen die austenitische Phase zunächst stabilisiert ist, d. h. die einen hohen Mn- und/oder C-Anteil aufweisen.

Beiden Varianten I und II ist gemeinsam, dass die durch die Wärmebehandlung bereitgestellten Elemente in das Innere des Implantatkörpers eindiffundieren, bevor sie Verbindungen mit Fe bilden, oder aus dem Inneren herausdiffundieren müssen. Thermodynamisch bedingt muss diese Diffusion einmal zum Stillstand kommen, da sich das Gitter mit den Einlagerungsatomen sättigt und beim Überschreiten der Löslichkeitsgrenze Fe-Verbindungen bildet bzw. das Gitter umklappt. Folglich bleibt bei einer Beschränkung des Elementangebots bzw. der Behandlungszeit sowie der entsprechenden Temperaturwahl die Dicke der oberflächennahen Grenzschicht, in der die Gefügeveränderung stattfindet, beschränkt.

### 1. Aufkohlen

In einem bevorzugten Ausführungsbeispiel des Verfahrens wird während der Wärmebehandlung gemäß Schritt b) der Gehalt an Kohlenstoff in dem Gefüge der oberflächennahen Grenzschicht durch Aufkohlen erhöht, wobei die Grenzschicht vorzugsweise eine Dicke von maximal 20 µm, besonders bevorzugt von maximal 10 µm aufweist.

In einer derartigen oberflächennahen Zone, welche mit Kohlenstoff und Kohlenstoffverbindungen angereichert ist, entstehen Eigenspannungen, insbesondere bei der Verwendung von Stahlsorten wie C10, C15 oder C20. Hierbei ist der Kohlenstoffgehalt und die Ausdehnung der Grenzschicht so gewählt, dass der maximale Gesamtwert des KohlenstoffGehalts des Implantats eine Marke von 0,45 Gew.% nicht übersteigt, so dass die mechanischen Eigenschaften des Implantats, insbesondere die Duktilität durch die Veränderungen nahe der Oberfläche nicht beeinträchtigt werden.

Die in der Grenzschicht des Implantatkörpers entstehenden Zugeigenspannungen begünstigen beim Dilatieren des Implantats die Entstehung von Rissen in der Oberfläche, welche wiederum die Degradation begünstigen. Ferner bewirken die permanent vorhandenen pulsativen Belastungen im weiteren Verlauf der Anwendung des Implantats zusätzlich eine spannungsrisskorrosionsbasierte Degradation. Die sich in das Substrat des Implantats ausbreitenden Risse führen jedoch nicht zu einem Versagen des gesamten Implantats (bei einem Stent beispielsweise zu einem Stegbruch), da die Risse durch die weniger rissempfindliche, zähe Matrix des Grundwerkstoffs, welche weniger Kohlenstoff enthält, aufgehalten werden. Die Korrosion besitzt hierdurch den Charakter einer Oberflächenkorrosion.

Die mittlere Konzentration von Kohlenstoff in der oberflächennahen Grenzschicht beträgt nach dem Aufkohlen 0,5 Gew.% bis 2 Gew.%, besonders bevorzugt 0,8 Gew.% bis 1,3 Gew.%.

Insbesondere werden im behandelten Teil des Implantatkörpers in dem angegebenen Bereich des Kohlenstoff-Gehalts die erforderlichen mechanischen Eigenschaften des Implantats, beispielsweise Rp_{0,2} von mindestens 300 MPa und A₅ von mindestens 15% erreicht. Hierbei stellt die Größe Rp_{0,2} die Dehngrenze dar, welche eine bestimmte werkstoffabhängige Spannung (hier: 300 MPa) angibt, die zu einer plastischen Verformung von 0,2% führt. Die sogenannte 0,2%-Dehngrenze wird auch als technische Elastizitätsgrenze bezeichnet. Der Parameter A₅ bezeichnet einen Materialkennwert, der die bleibende Verlängerung der Probe nach dem Bruch, bezogen auf die Anfangsmesslänge, angibt. Diese charakterisiert die Verformungsfähigkeit des Werkstoffs. Die Kenngröße A₅ wird berechnet aus dem Verhältnis (in Prozent) aus der bleibenden Längenänderung einer Probe im Zugversuch bezogen auf ihre Anfangsmesslänge.

Bei diesem Ausführungsbeispiel der vorliegenden Erfindung wird die Kohlenstoff-Konzentration so eingestellt, dass die Zusammensetzung des Gefüges im Perlit-Bereich des Eisen-Kohlenstoff-Diagramms liegt. Da der Perlit ein Phasengemisch aus Zementit und Ferrit darstellt, wirkt der Zementit als kathodischer Bereich eines Lokalelements. Der benachbart angeordnete Ferrit korrodiert somit beschleunigt. Die korrosionsbeständigeren Zementitlamellen (Fe₃C) bleiben eine Zeit lang inselartig stehen, so dass die Oberfläche durch selektive Korrosion zerklüftet wird.

Es ist ebenfalls von Vorteil, wenn der Implantatkörper nach dem Aufkohlen derart abgekühlt wird, dass in der oberflächennahen Grenzschicht zusätzliche Eigenspannungen entstehen. Diese erzeugen beim Dilatieren ebenfalls Risse in der Oberfläche, welche als Ausgangspunkt der Degradation wirken können.

In einem weiteren Ausführungsbeispiel weist der Körper des Implantats in der durch die Wärmebehandlung entstandenen Grenzschicht einen Anteil von höchstens 40 Gew.% Zementit auf.

### 2. Borieren

In einem weiteren vorteilhaften Ausführungsbeispiel wird während der Wärmebehandlung gemäß Schritt b) der Gehalt an Bor in dem Gefüge der oberflächennahen Grenzschicht durch Borieren erhöht, wobei die Grenzschicht vorzugsweise eine Dicke 5 µm bis 300 µm aufweist. Die Schichtdicke ist u.a. vom Kohlenstoffgehalt der Ausgangszusammensetzung des entsprechenden Teils des Implantatkörpers abhängig.

Durch Borieren bildet sich eine an der Oberfläche angeordnete Boridschicht aus, welche im Fall von überwiegend Eisen als Material des Implantatkörpers FeB und Fe₂B enthält. Diese Bor-Verbindungen wachsen zahn- oder stängelartig senkrecht zur Oberfläche des Implantatkörpers in das Gefüge hinein und sind gut in dem Gefüge verankert. Sie bewirken eine Volumenzunahme der behandelten Randzone von etwa 25%. Insbesondere bei Beanspruchung in alkalischen Medien führt eine derartige Grenzschicht zu einer Abnahme des Korrosionswiderstandes bei günstigen mechanischen Eigenschaften. Insbesondere wird abhängig von der Ausgangszusammensetzung des Implantatkörpers, d. h. abhängig von dessen Fe-Gehalt, eine einphasige Grenzschicht aus Fe₂B angestrebt. Die mit einer derartigen Grenzschicht erreichbare Härte beträgt bei einer einphasigen Schicht aus FeB etwa 2100 HV, bei einer Schicht aus Fe₂B etwa 2000 HV. Der Gehalt von Bor in FeB beträgt 16,23 Gew.%, in Fe₂B 8,83 Gew.%. Bei einem geringen Anteil an Fe in der Ausgangszusammensetzung wird beim Borieren ein größerer Anteil an Bereichen erzeugt, die nicht Fe₂B enthalten.

Beim Borieren werden zudem Elemente wie Silizium und Kohlenstoff unter die Boridschicht abgedrängt, wodurch sich eine nicht austenitisierbare ferritische Schicht bilden kann, die als Lokalelement ebenfalls eine Beschleunigung der Korrosion bewirken kann. Im Falle lokal erhöhter Mangangehalte können Inseln von Restaustenit in der ferritischen Oberflächenmatrix entstehen. Die meisten metallischen Legierungselemente sind dagegen in der Boridschicht löslich. Dies gilt insbesondere für Mangan, dessen Anteil in der Boridschicht weitgehend dem des Grundwerkstoffs entspricht, und Chrom, bei dem man in der Boridschicht leicht höhere Anteile findet. Daher müsste die Zusammensetzung der Boridschicht eigentlich mit (Fe, M)B bzw. (Fe, M)₂B charakterisiert werden, wobei M ein oder mehrere, in der Schicht lösliche Elemente bezeichnet.

Die mittlere Konzentration von Bor in der oberflächennahen Grenzschicht beträgt nach dem Borieren 1 Gew.% bis 20 Gew.%, vorzugsweise 7 Gew.% bis 10 Gew.%.

Vorzugsweise werden die Implantate über einen Zeitraum von etwa 15 Minuten bis etwa 6 Stunden boriert. Das Borieren findet vorzugsweise in einem Temperaturbereich zwischen etwa 850°C und 1.000°C statt. Dabei beeinflussen die Verfahrensparameter des Borierens, insbesondere die Temperatur und die Borierdauer, die Haftfestigkeit und Korrosionsanfälligkeit der oberflächennahen Grenzschicht (Borierzone). Bei höheren Temperaturen bilden sich längere und unregelmäßigere Zahn- oder Stängelstrukturen, die, wie oben beschrieben von der Oberfläche in den Festkörper hineinwachsen. In diesem Fall wird die Korrosionsanfälligkeit des Implantats weiter erhöht, da nach dem Herauslösen der Stängelstrukturen aus der Matrix eine sehr zerklüftete Oberfläche stehen bleibt, die aufgrund ihrer hohen Oberfläche besser weiter degradieren kann. Bei niedrigeren Temperaturen und kürzeren Borierzeiten werden die Zahn- oder Stängelstrukturen regelmäßiger und kleiner. Insgesamt hat sich gezeigt, dass die Anwendung von höheren Temperaturen und entsprechend kürzeren Verfahrenszeiten günstiger ist, da die Diffusionstiefe des Bors bei kürzeren Verfahrenszeiten begrenzt bleibt und nicht der gesamte Implantatquerschnitt boriert wird. Ein Borieren des gesamten Querschnitts hätte eine zu große Gesamtversprödung des Implantats zur Folge, so dass beim Dilatieren durchgehende Brüche im Implantat, zum Beispiel bei Stents Stegbrüche, entstehen können.

### 3. Nitrocarburieren

In einem weiteren Ausführungsbeispiel wird während der Wärmebehandlung gemäß Schritt b) der Gehalt an Kohlenstoff und Stickstoff in dem Gefüge der oberflächennahen Grenzschicht durch Nitrocarburieren erhöht, wobei die Grenzschicht vorzugsweise eine Dicke von maximal 150 µm aufweist. Der besondere Vorteil einer solchen Vorgehensweise besteht darin, dass die so behandelten Implantate formbeständig sind und eine hohe Maßhaltigkeit aufweisen, da beim Nitrieren keine Gefügeumwandlung stattfindet.

Durch das Nitrocarburieren entstehen Nitride, insbesondere das γ'-Nitrid oder weiter bevorzugt das ε-Carbonitrid welche ebenfalls benachbarte Zonen (Lokalelemente) bilden, die sich hinsichtlich ihrer Position in der elektrochemischen Spannungsreihe und ihrer Sprödigkeit von den umliegenden Bereichen unterscheiden. Dadurch werden anodische und kathodische Teilbereiche gebildet und Verspannungen erzeugt. Die Umgebung der Nitride korrodiert somit schneller. Dies führt zu einer verstärkten Zerklüftung der Oberfläche, die eine weitere beschleunigte Korrosion begünstigt.

Das Nitrocarburieren kann bspw. bei einer Temperatur im Bereich von 570°C bis 580°C vorzugsweise in einem Gasgemisch erfolgen, welches Stickstoff (z. B. Ammoniak) und Kohlenstoff (z. B. Kohlendioxid, Methan, Ethan, Propan) abgeben kann.

In einem besonders bevorzugten Ausführungsbeispiel beträgt die mittlere Konzentration in der oberflächennahen Grenzschicht nach dem Nitrocarburieren 0,5 Gew.% bis 7 Gew.%, bevorzugt 0,5 Gew.% bis 4 Gew.%, besonders bevorzugt 0,5 Gew.% bis 2 Gew.% Kohlenstoff und 0,5 Gew.% bis 17 Gew.%, bevorzugt 5 Gew.% bis 12 Gew.% Stickstoff. Hierbei ist festzustellen, dass Nitride eine bestimmte Löslichkeit für Kohlenstoff besitzen. So kann das γ'-Nitrid bis zu 0,2 Gew.% Kohlenstoff aufnehmen, das ε-Nitrid sogar bis zu 4 Gew.%. Durch die Regelung der Nitrierkennzahl oder Nitrierkenngröße N lassen sich das Wachstum und die Zusammensetzung der Nitrierschicht gezielt beeinflussen. Bei der y'-Phase schwankt die stöchiometrische Zusammensetzung nur geringfügig. Beispielsweise kann die Schwankung an Stickstoff in Masse-% zwischen 5,7 und 6, 1 % N schwanken. Die ε-Phase kann dagegen beispielsweise zwischen 5,6 und 11 % N enthalten.

Nitrocarburierschichten setzen sich in der Regel aus zwei Zonen, nämlich einer innen liegenden Zone (innere Zone) und einer außen liegende Zone, welche die Oberfläche des Implantats in dem behandelten Bereich bildet, zusammen. Beide Zonen bilden die oberflächennahe Grenzschicht, deren N- und ggf. auch C-Gehalt durch Nitrocarburieren erfindungsgemäß geändert wird.

Die innere Zone, welche die Diffusionsschicht darstellt, kann durch die Bildung von Nitridnadeln charakterisiert werden. Die Dicke der Diffusionsschicht hängt von verschiedenen Parametern (hinsichtlich des Werkstoffs des Implantatkörpers insbesondere von den Legierungsbestandteilen, aber auch von der Temperatur, der Nitrocarburierzeit, der Gaszusammensetzung bei der Gasnitrocarburierung, die insbesondere durch die sogenannte Nitrierkenngröße N (Ammoniakanteil in der Gasmischung) ausgedrückt werden kann.

Die äußere, 5 µm bis 30 µm dicke nichtmetallische Zone setzt sich im Wesentlichen aus γ'-Nitriden (Fe4N), ε-Nitriden (Fe2...3N) und gegebenenfalls Carbonitriden (FexCyNz) zusammen. Bei legierten Stählen finden sich außerdem Nitride und Carbonitride der Legierungselemente. Der äußerste, in Richtung der Oberfläche des Implantats angeordnete Bereich der außen liegenden Schicht kann als Porensaum ausgebildet sein.

Insgesamt ist es bei den Verfahren vorteilhaft, dass sich mit den Verfahrensparametern Prozesstemperatur, Partialdruck, Zusammensetzung des Prozessgases und Prozesszeit das gewünschte Eigenschaftsprofil des Implantats einfach einstellen lässt. Dies gilt insbesondere für die Degradationsgeschwindigkeit, aber auch für die Verschleißfestigkeit des Implantats gegen Abrasion.

Ein weiterer Vorteil des Verfahrens besteht darin, dass durch die erfindungsgemäß angewendete Wärmebehandlung in dem Implantat im Bereich der oberflächennahen Grenzschicht Poren erzeugt werden. Diese können einerseits als Vehikel für den Transport von pharmazeutisch aktiven Substanzen und andererseits zur Erleichterung der Verankerung des Implantats in dem umliegenden Gewebe, bspw. in Knochen, dienen.

Ein weiterer Vorteil der durch das Verfahren erzeugten oberflächennahen Grenzschicht besteht darin, dass sie eine erhöhte Beständigkeit des Implantats gegen abrasiven Verschleiß bzw. gegen den Reibanteil bei der Reibkorrosion bewirkt. Insbesondere bei Anwendungen, die unvermeidlich die Reibung des Implantats mit sich selbst, mit einem anderen Implantat oder mit einem harten Gewebe (Knochen) bedingen (bspw. beim Eindrehen einer Knochenschraube), ist diese Eigenschaft des mit dem Verfahren hergestellten Implantats von Vorteil.

### 4. Entkohlen

In einer weiteren Ausführungsform wird bei der Wärmebehandlung gemäß Schritt b) der Gehalt an Kohlenstoff im Gefüge der oberflächennahen Grenzschicht des Implantats durch Entkohlen verringert, wobei die Grenzschicht vorzugsweise eine Dicke von maximal 50 µm aufweist.

Diese erfindungsgemäße Lösung kommt insbesondere bei Implantaten zum Einsatz, welche aus einem derart mit Mangan und Kohlenstoff legierten Eisenbasiswerkstoff bestehen, dass die austenitische Phase stabilisiert wird. Beispielsweise kann das Material des Implantatkörpers eine Eisenbasislegierung mit 0,7 Gew.% Kohlenstoff und 12 Gew.% Mangan enthalten. Durch eine Wärmebehandlung in oxidierender Atmosphäre (bspw. in reinem Sauerstoff oder Luft) verbrennt der Kohlenstoff in der oberflächennahen Grenzschicht des Implantats. Hierdurch reduziert sich der Kohlenstoff-Gehalt in der Grenzschicht und beträgt bspw. in einer Tiefe von 5 µm nur noch 0,1 Gew.%. Je nach Verfahrensführung kann diese Randentkohlung bis in eine Tiefe von 50 µm reichen. Dort beträgt der Kohlenstoffgehalt dann noch 0,06 Gew.%. Gemittelt über die gesamte Grenzschicht beträgt der Kohlenstoffgehalt 0,1 Gew.% bis 0,3 Gew.%, bevorzugt 0,15 Gew.% bis 0,25 Gew.%.

Wenn ein so behandeltes Implantat nachfolgend plastisch verformt wird, bspw. beim Dilatieren, so wird die oberflächennahe Grenzschicht mechanisch stärker verformt als die Bereiche im Inneren des Implantats. In dem oberflächennahen Grenzbereich, der weniger Kohlenstoff enthält, ist durch die Verringerung des Kohlenstoffgehalts das Austenitgefüge metastabil und es entsteht ein spannungsinduzierter Martensit. Dieser zeichnet sich durch eine höhere mechanische Festigkeit und eine geringere Bruchdehnung aus. Der spannungsinduzierte Martensit neigt beim Dilatieren und auch bei weiterer mechanischer Beanspruchung nach dem Dilatieren, bspw. durch eine Gefäßbewegung, zur Rissbildung. In den Rissen entstehen beim Kontakt mit Körperflüssigkeit Zonen, in denen Chloridionen, welche in der Körperflüssigkeit vorhanden sind, stärker adsorbiert werden. Durch den geringeren Stoffaustausch wird die Passivität im Spalt aufgehoben. Die sich bei der Spaltkorrosion bildenden Korrosionsprodukte hydrolysieren und der pH-Wert sinkt lokal. Dies bewirkt eine weitere Beschleunigung der Korrosion. Diese greift damit zunehmend auch rissferne Bereiche an.

Die oben beschriebenen Korrosionsvorgänge werden durch mechanische Belastungen des Implantats begünstigt. Im Fall eines Stents ist eine derartige mechanische Belastung die pulsative Belastung des Stents durch die Gefäßkontraktion. Bei orthopädischen Implantaten kann eine mechanische Belastung durch eine durch die Schrittfrequenz hervorgerufene wechselnde Zug-Druckbelastung (z. B. bei einer filigranen Knochenschraube oder einer distalen fibularen Tibiaplatte für Kleinkinder) hervorgerufen werden. Diese mechanische Belastung fördert die Spannungsrisskorrosion. Überkritische Risse, welche plötzlich und im schlimmsten Fall zu einem Versagen des Implantats führen würden, werden dadurch verhindert, dass unterhalb der oberflächennahen Grenzschicht, d. h. im Inneren des Implantats, ein zäher, rissunempfindlicher austenitischer Bereich vorliegt, welcher die Rissenergie im kubisch-flächenzentrierten austenitischen Gitter akkumuliert und teilweise auch dissipiert. Die in diesem Bereich gestoppten Risse bewirken jedoch erneut eine Spaltkorrosion und der Korrosionsprozess setzt sich in ähnlicher Weise beschleunigt bis zur gewünschten Selbstauflösung des Implantats fort.

Die mittlere Konzentration von Kohlenstoff in der oberflächennahen Grenzschichtbeträgt nach dem Entkohlen 0,1 Gew.% bis 0,3 Gew.%. Der Mangan-Gehalt liegt vorzugsweise bei 10 Gew.% bis 19,5 Gew.%.

Eine weitere Beschleunigung der Degradation kann dadurch erreicht werden, dass der durch Entkohlen behandelte Teil des Implantatkörpers rasch abgekühlt wird. Dies bedeutet, dass der Teil des Implantatkörpers mit Hilfe eines vorzugsweise flüssigen Kühlmediums (außer Luft) abgeschreckt wird. Durch das schnelle Abkühlen wird eine rasche Gefügeumwandlung bewirkt, so dass aufgrund der unterschiedlichen thermischen Ausdehnungskoeffizienten zwischen Austenit und Martensit Härterisse erzeugt werden. Diese Risse bilden ebenfalls einen Angriffspunkt für die oben beschriebene Korrosion. Beispielsweise kann das Implantat in salzhaltiger Eiswasserlösung mit einer Temperatur von -10°C oder in flüssigem Stickstoff mit einer Temperatur von -196°C ausgehend von der Austenitisierungstemperatur abgeschreckt werden.

### 5. Zusätzliche Beschichtung und tribochemische Behandlung

Es ist weiterhin von Vorteil, dass nach der Wärmebehandlung gemäß Schritt b) eine Beschichtung des Implantatkörpers zumindest auf einem Teil seiner Oberfläche mit einer pharmazeutisch aktiven Substanz, insbesondere eingebettet in ein Polymer, beispielsweise ein Polylactid, ein Polyglycosid oder ein Copolymer aus diesen, besonders bevorzugt PLLA oder PLGA, oder ein Blend aus den genannten Polymeren erfolgt. Diese zusätzliche Beschichtung kann für Implantate angewendet werden, welche entweder nach Variante I oder II der Wärmebehandlung hergestellt wurden.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutika zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, bestehen.

Insbesondere vorteilhaft ist eine Beschichtung mittels eines Polymers, beispielsweise ein Polylactid, ein Polyglycosid oder ein Copolymer aus diesen, besonders bevorzugt PLLA oder PLGA, oder ein Blend aus den genannten Polymeren, welches die pharmazeutisch aktive Substanz enthält, da so die bei der Degradation des Polymers erzeugte Verringerung des pH-Werts im Bereich der Implantatoberfläche einen zusätzlichen Beschleunigungsfaktor für die Korrosion, insbesondere bei einem Implantat mit einer Eisenlegierung, darstellt.

Alternativ oder zusätzlich (nach der Wärmebehandlung und vor einer Beschichtung des Implantatkörpers mit einer pharmazeutisch aktiven Substanz) kann der Implantatkörper in einem weiteren Ausführungsbeispiel zumindest auf einem Teil seiner Oberfläche tribochemisch mittels Strahlteilchen behandelt werden.

Unter der tribochemischen Behandlung wird in der vorliegenden Anmeldung eine mechanische und ggf. chemische Oberflächenbehandlung mittels Beschuss bzw. Bestrahlen des behandelten Teils der Körperoberfläche durch Strahlteilchen (Partikel) verstanden. Das Verfahren wird auch als Partikelstrahlen bezeichnet. Die Strahlteilchen sind dabei Teilchen bestehend aus einem oder mehreren Materialien mit einer mittleren Partikelgröße im Nano- oder Mikrometerbereich. Hierbei wird die Partikelgröße unter Anwendung eines Rasterelektronenmikroskops (bei Partikelgrößen kleiner oder gleich 3 µm) oder eines Lichtmikroskops (bei Partikelgrößen größer als 3 µm) ermittelt. Die elektronen- oder lichtmikroskopischen Aufnahmen werden vermessen und aus den Messwerten, beispielsweise durch Mittelwertbildung (arithmetisches Mittel), die mittlere Partikelgröße bestimmt. Die Strahlteilchen bestehen demnach nicht aus einzelnen Atomen, Ionen oder Verbindungen sondern jeweils aus einer Gruppe oder einem Gefüge von Atomen, Ionen- oder Molekülverbindungen. Die tribochemische Behandlung wird in der Regel bei Raumtemperatur, d. h. im kalten Zustand durchgeführt. Im Folgenden wird hauptsächlich auf das Bestrahlen mittels Strahlteilchen als tribochemische Behandlungsmethode Bezug genommen.

Die tribochemische Behandlung mindestens eines Teils der Körperoberfläche bewirkt besonders einfach und kostengünstig mittels der Strahlteilchen die Erzeugung einer zusätzlichen Oberflächenrauhigkeit und/oder einer Beschichtung jeweils auf dem behandelten Teil der Körperoberfläche. Dabei wird die durch die Wärmebehandlung ausgebildete Oberfläche hinsichtlich ihrer chemischen Zusammensetzung, des Verfestigungsgrades und/oder der Oberflächenmorphologie derart zielgerichtet verändert, dass weitere eine Veränderung des Degradationsverhaltens des behandelten Implantats bewirkt wird. Die bei der tribochemischen Behandlung entstehenden Veränderungen der Oberfläche hängen insbesondere ab von der Art, der Größe, der Form und der chemischen Zusammensetzung der Strahlteilchen, von dem Strahldruck und dem Abstand zwischen der Strahldüse und dem Implantatkörper sowie dem Material des Implantatkörpers.

Insbesondere werden für die tribochemische Behandlung Strahlteilchen verwendet, welche mindestens ein Salz aufweisen, vorzugsweise eine Verbindung aus der Gruppe enthaltend NaCl, CaCl, MgCl₂, insbesondere trockengelagertes MgCl₂.

Die Strahlteilchen mit mindestens einem Salz werden bei einem bestimmten, vorgegebenen Druck auf die Oberfläche des Implantats gestrahlt. Der in vorteilhafter Weise anzuwendende Druckbereich liegt dabei zwischen 0,5 und 5,0 bar. Die Behandlungsdauer liegt im Bereich von 0,5 bis 30 min. Die Aufbringung eines Druckes bei der Bestrahlung bewirkt neben der Aufrauung der Körperoberfläche auch ein teilweises Adhärieren der aufgestrahlten Salze und ggf. weiterer Strahlteilchen auf der Körperoberfläche. Dabei können die Strahlteilchen, welche die korrosiv wirkenden Salze aufweisen, auch mit Strahlteilchen aus inertem Hartmaterial, vorzugsweise eine Verbindung aus der Gruppe enthaltend Oxide, insbesondere Al₂O₃, SiO₂, ZrO₂, MgO, Karbide, insbesondere TiC, SiC, B₄C, B-BN, (Berylliumcarbid), Oxikarbide, Nitride, insbesondere TiN, kubisches Bornitrid (c-BN, β-BN, "Borazon"), Si₃N₄, AlN und TiAlN, Karbonitride, natürlicher oder synthetischer Diamant sowie Bor, vermischt werden. Die auf der Oberfläche des Implantatkörpers adhärierten Salze bewirken eine beschleunigte Korrosion der Oberfläche.

Bei diesem Ausführungsbeispiel ist auch ein ausschließlicher Einsatz von korrosiv wirksamen Salzen als Strahlteilchen möglich. Insbesondere wird trocken gelagertes MgCl₂ als körniges Salz definierter Körnung als Strahlmittel ohne Zusatz von weiteren abrasiv wirkenden Hartstoffen verwendet. Trocken gelagertes MgCl₂ sollte aus dem Grund verwendet werden, da sonst Verklumpungsgefahr durch starke Hygroskopie des Materials besteht.

MgCl₂-Teilchen bewirken aufgrund ihrer geringen Härte keine wesentliche mechanische Oberflächenveränderung, d. h. keine wesentliche Oberflächenaufrauung. Allerdings adhäriert ein großer Anteil der MgCl₂-Teilchen an der Oberfläche des Implantats. Bei anschließender Lagerung des so behandelten Implantats unter extrem trockener Atmosphäre und unter Luftabschluss wird eine Korrosion der Oberfläche zunächst verzögert. Erst zum Zeitpunkt der Implantation und dem Kontakt des Implantats, beispielsweise eines Stents, mit der Körperflüssigkeit beginnt das anhaftende MgCl₂ stark korrosiv zu wirken, so dass eine beschleunigte Degradation erfolgt.

Bei nicht ausschließlich trockener Lagerung verbleiben die MgCl₂-Partikel temporär bis zu ihrer Auflösung in der tribochemisch erzeugten Submikroaufrauung, beschleunigen die Korrosion bei Anwesenheit von Luftfeuchtigkeit und vergrößern somit die Abmessungen der Kavität der Submikroaufrauung.

In einem weiteren bevorzugten Ausführungsbeispiel erfolgt nach einer tribochemischen Behandlung eine Auslagerung des tribochemisch behandelten Implantatkörpers bei erhöhter Luftfeuchtigkeit, insbesondere bei einer Luftfeuchtigkeit größer als 80%, und/oder bei erhöhter Temperatur, insbesondere bei einer Temperatur von mehr als 50°C.

Durch die angegebene Nachbehandlung des Implantatkörpers, insbesondere nach einer tribochemischen Behandlung durch Beschuss mit Strahlteilchen, die das mindestens eine Salz enthalten, wird eine besonders beschleunigte Korrosion der Körperoberfläche erreicht. Diese Nachbehandlung, insbesondere die Auslagerung bei erhöhter Luftfeuchtigkeit, bewirkt die Entstehung von ganz spezifischen, lokal qualitativ unterschiedlichen Lochkorrosionseffekten.

Die durch die tribochemische Behandlung entstehende Oberflächenstruktur kann zusammenfassend mittels folgenden morphometrischen und chemischen Eigenschaften charakterisiert werden:
- Mikroaufrauung im Rauheitsbereich Rz von mehreren µm,
- Submikroaufrauung (Kavernen) im Rauheitsbereich Rz von < 1 µm,
- chemische Modifizierung durch adhärierte leitfähige Strahlpartikel und/oder Salze und
- eine im Vergleich zum Grundwerkstoff erhöhte Versetzungsdichte bis in eine Tiefe des Implantatkörpervolumens von bis zu etwa 5 µm ausgehend von der Körperoberfläche.
Diese Oberflächenstruktur führt zu einer weiteren Beschleunigung der Degradation des Implantats.

### 6. Implantat

Die obige Aufgabenstellung wird ferner gelöst durch ein Implantat erhältlich durch ein oben beschriebenes Verfahren. Ein derartiges Implantat weist die oben im Zusammenhang mit dem erfindungsgemäßen Herstellungsverfahren angegebenen Vorteile auf.

Ferner wird die obige Aufgabenstellung durch ein Implantat gelöst, bei dem in dem Gefüge der oberflächennahen Grenzschicht mindestens eines Teils des Implantatkörpers gegenüber der jeweiligen Konzentration im restlichen Gefüge
- ein erhöhter Kohlenstoffanteil, vorzugsweise in einer Konzentration von 0,5 Gew.% bis 2 Gew.%, besonders bevorzugt in einer Konzentration von 0,8 Gew.% bis 1,3 Gew.%, oder
- ein erhöhter Boranteil, vorzugsweise in einer Konzentration von 1 Gew.% bis 20 Gew.%, besonders bevorzugt in einer Konzentration von 7 Gew.% bis 10 Gew.%, oder
- ein erhöhter Kohlenstoff- und Stickstoffanteil, vorzugsweise in einer Konzentration von Kohlenstoff von 0,5 Gew.% bis 7 Gew.%, besonders bevorzugt in einer Konzentration von 0,5 Gew.% bis 4 Gew.%, insbesondere von 0,5 Gew.% bis 2 Gew.%, und von Stickstoff von 0,5 Gew.% bis 17 Gew.%, besonders bevorzugt von 5 Gew.% bis 12 Gew.%, oder
- ein verringerter Kohlenstoffanteil, vorzugsweise in einer Konzentration von 0,1 Gew.% bis 0,3 Gew.%, besonders bevorzugt in einer Konzentration von 0,15 Gew.% bis 0,25 Gew.%,
vorliegt. Auch ein solches erfindungsgemäßes Implantat weist die oben beschriebenen Vorteile auf.

Wie bereits oben dargestellt ist es von Vorteil, dass ein solches Implantat in und/oder nahe der Oberfläche Poren aufweist. Diese dienen insbesondere zur Aufnahme einer pharmazeutisch aktiven Substanz bzw. zur besseren Verankerung des Implantats im Gewebe des behandelten Körpers.

Wie oben erläutert ist es weiter von Vorteil, wenn die Oberfläche des Implantatkörpers zumindest teilweise eine Beschichtung aufweist, welche eine pharmazeutisch aktive Substanz enthält.

Weiter bevorzugt ist, dass der Körper des Implantats überwiegend Eisen aufweist, vorzugsweise mehr als 80 Gew.%, besonders bevorzugt mehr als 99 Gew.% Eisen, insbesondere in einer Legierung.

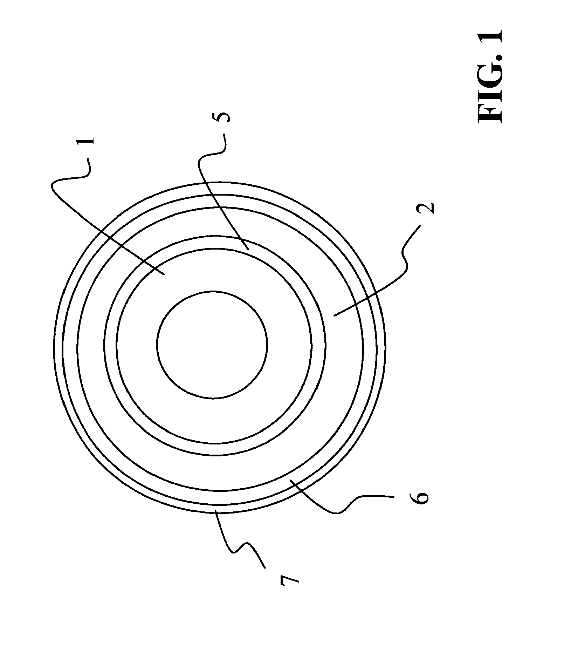

Das erfindungsgemäße Implantat wird nachfolgend anhand von Beispielen und einer Figur 1 erläutert. Dabei bilden alle in der Figur 1 gezeigten und beschriebenen Merkmale den Gegenstand der Erfindung, unabhängig von ihrer Zusammenfassung in den Ansprüchen oder deren Rückbezüge. Für alle nachfolgend erläuterten Beispiele gilt, dass durch die Wärmebehandlung mindestens eine Verringerung der Degradationszeit auf 75%, in einigen Fällen auf 60% der Degradationszeit gegenüber der Degradationszeit erreicht wurde, die bei dem jeweiligen Implantat vorliegt, wenn keine Wärmebehandlung durchgeführt wurde. Die Degradationszeit kann durch eine nachfolgend ausgeführte tribochemische Behandlung weiter verringert werden.

### 1. Beispiel (Variante I):

Ein Stahl, wie beispielsweise C10, C15 oder C20, wird mittels Aufkohlen behandelt und derart abgekühlt, dass die geforderten mechanischen Eigenschaften (Rp_{0,2} mindestens 300 MPa und A₅ mindestens 15%) erreicht werden. Es wird eine Aufkohlungstiefe von ca. 5 µm bis 20 µm eingestellt. Der Gesamtgehalt an Kohlenstoff in dem behandelten Implantat überschreitet einen Anteil von 0,45 Gew.% nicht.

Die aufzukohlenden Teile werden in kohlenwasserstoffhaltige, gasförmige Atmosphären eingesetzt. In einer Wärmebehandlungsapparatur (Aufkohlungsofen) wird eine Aufkohlungsatmosphäre erzeugt, welche aus einem leicht reduzierenden Trägergas und einem geregelt zugeführten Anreicherungsgas besteht. Das Trägergas besteht üblicherweise aus 20Vol.% Kohlenmonoxid, 40Vol.% Wasserstoff und 40Vol.% Stickstoff. Das Anreicherungsgas ist ein Kohlenwasserstoff, z. B. Methan oder Propan. Die Prozesstemperatur beträgt zwischen 850°C und 900°C. Die Behandlungszeit liegt bei den genannten Aufkohlungstiefen von maximal 20 µm bei maximal 10 Minuten. Anschließend werden die Implantate der Ofenkammer entnommen und an ruhender Luft abgekühlt.

### 2. Beispiel (Variante I): (Vergleich)

Körper von Implantaten, beispielsweise Stentkörper, bestehend aus den Stählen C10, C15 oder C20 werden in einen Gasnitrierofen verbracht. Als Stickstoff abgebendes Trägergas kommt Ammoniak (NH₃) zum Einsatz. Auf Grund des filigranen Aufbaus des Stentkörpers werden keine flüssigen oder festen Stickstoffträger verwendet.

Beim Gasnitrieren spaltet sich laufend während der gesamten Wärmebehandlungsdauer Ammoniak (NH₃) in die Bestandteile atomarer Stickstoff und Wasserstoff auf Diese Vorgänge geschehen unter Einwirkung von Temperatur und der katalytischen Wirkung der Eisenoberfläche. Der dann vorliegende atomare Stickstoff diffundiert in die Implantatoberfläche ein. Die Nitriertemperatur beim Gasnitrieren liegt bei ca. 500°C bis 530°C. Die Nitrierzeit beträgt 10 Minuten bis 20 Minuten. Anschließend werden die Implanate der Ofenkammer entnommen und an ruhender Luft abgekühlt.

### 3. Beispiel (Variante I):

Der Stahl C15 hat folgende Ausgangs-Zusammensetzung (Angaben in Gew.%):
C (0,12- 0,18)
Si (max. 0,40)
Mn (0,30 - 0,60)
P (max. 0,045)
S (max. 0,045)

Ein Stentkörper aus C 15-Stahl mit der angegebenen Zusammensetzung wird nitrocarburiert. Das Nitrocarburieren ist eine Kombination aus Aufkohlen und Nitrieren. Für Stents ist es von Vorteil, wenn Nitrocarburieren in der Gasphase durchgeführt wird. Bei der Behandlung in relativ hochviskosen Salzschmelzen würden sich Salzkrusten zwischen den Stentstrukturen bilden und somit ein Eindiffundieren des Stickstoffs und des Kohlenstoffs verhindern.

Die Temperatur für die Wärmebehandlung während des Nitrocarburierens liegt im Bereich zwischen 700°C bis 800°C. Die Gasatmosphäre ist vergleichbar mit dem Beispiel 1 (Variante I) allerdings mit Ammoniakzusatz. Es ergibt sich 20 Vol.% CO, 30 Vol.% H₂, 10 Vol.% NH₃ und 40Vol.% N2. Die Behandlungszeit beträgt auch hier ca. 10 Minuten. Anschließend werden die behandelten Stentkörper der Ofenkammer entnommen und an ruhender Luft abgekühlt.

### 4. Beispiel (Variante II):

Dieses Beispiel betrifft einen Stent aus einer degradierbaren Eisenbasislegierung mit 12 Gew.% Mn und anfänglichen 0,7 Gew.% C.

Als Ausgangsmaterial dient ein Rohr, aus dem ein Stent hergestellt werden soll, aus einer Eisenbasislegierung mit 12 Gew.% Mn und 0,7 Gew.% C mit einem Außendurchmesser von 2,0 mm und einer Wanddicke von 120 µm. Die Zusammensetzung des Materials im Ausgangszustand führt zu einem austenitischem Gefüge. Das Rohrmaterial weist im Zugversuch eine Mindestzugfestigkeit Rₘ von 800 MPa bei einer Mindestbruchdehnung A₅ von 15% auf.

Aus diesem Rohr wird mittels Laserschneiden ein Stent für koronare Anwendungen geschnitten. Nach den üblichen Verfahrensschritten wie Entgraten, Korundstrahlen und das für die Kantenverrundung erforderliche Elektropolieren, ist das Gefüge des Rohrs immer noch austenitisch. Der so entstandene Stentkörper ist eine rohrförmige Stegstruktur. Der Querschnitt eines einzelnen Steges des Stentkörpers beträgt nach Abschluss der genannten Schritte etwa 100 µm x 100 µm. Anschließend erfolgt ein Glühen (Entkohlen) in einer Sauerstoffatmosphäre bei 900°C bis 1000°C über einen Zeitraum von 30 Minuten. Dabei reduziert sich der C-Gehalt in der oberflächennahen Grenzschicht in Abhängigkeit von der Tiefe. Die Abkühlung erfolgt durch plötzliche Zwangsbelüftung des Ofenraums mit Stickstoff. Der Stent kühlt hierdurch innerhalb von 180 Sekunden auf 500°C ab.

Der durch die obige Wärmebehandlung erzeugte C-Gehalt beträgt in einer Tiefe von 5 µm nur noch 0,1%. Die Randentkohlung schreitet bis eine Tiefe von 40 µm fort. Hier beträgt der C-Gehalt dann noch 0,6%. Der Gefügezustand ist hier immer noch austenitisch.

Anschließend wird der Stent der Wärmebehandlungskammer entnommen, gecrimpt und in einen Katheter montiert.

Beim klinischen Einsatz wird dieser Stent mittels Ballon auf einen Enddurchmesser von 3,5 mm dilatiert. In den plastisch am stärksten verformten Zonen des Stents erfolgt in der Grenzschicht eine Phasenumwandlung von der austenitischen in die martensitische Phase. Die Umwandlungszone reicht bis in die Tiefe von 15 µm. Dort beträgt der C-Gehalt 0,2 Gew.%. Da die Randentkohlungseffekte über alle 4 Seiten der Stentstruktur (ehemalige Rohrinnenseite, ehemalige Rohraußenseite und jeweils zwei Laserschnittkanten) auftreten, liegt der ursprüngliche C-Gehalt von 0,7 Gew.% nur noch in einer Querschnittsbreite von 20 µm x 20 µm vor. Durch die Wärmebehandlung reduziert sich damit der C-Gehalt des gesamten Stents auf maximal 0,45 Gew.%.

### 5. Beispiel (Variante II):

Dieses Beispiel betrifft eine orthopädische Endoprothese aus einer degradierbaren Eisenbasislegierung mit 12 Gew.% Mn und anfänglich 0,7 Gew.% C.

Dieses Ausführungsbeispiel entspricht dem Beispiel 4, wobei anstelle des Stents eine Schraube zur Fragmentstabilisierung nach Schädelnahterweiterung bei Patienten mit Craniosynostosen verwendet wird. Eine derartige Endoprothese, bspw. eine Kortikalisschraube mit 1,5 mm Durchmesser und einer Gewindetiefe von 0,5 mm, wird einer Wärmebehandlung analog zu Beispiel 4 unterzogen. Die aus der Wärmebehandlung resultierende Gefügezusammensetzung entspricht der in Beispiel 4 beschriebenen.

Durch die im Schädelbereich fehlende pulsierende mechanische Belastung erfolgt bei einer derartigen Endoprothese keine Spannungsrisskorrosion. Dadurch degradiert diese Endoprothese etwas langsamer als der in Beispiel 4 dargestellte Stent. Die Schraube wird jedoch dennoch innerhalb von 12 bis 18 Monaten durch den Schädelknochen verstoffwechselt. Die Gefügeumwandlung findet bei diesem Ausführungsbeispiel ausschließlich im Bereich der Gewindeflanken statt. Die Oberflächen der Gewinde werden beim Einschrauben in das Innengewinde einer ebenfalls aus einer degradierbaren Eisenlegierung bestehenden Knochenplatte verquetscht und damit plastisch verformt. Diese Verformung führt zu der beschriebenen oberflächlichen Gefügeumwandlung. Das Ergebnis der Gefügeumwandlung wird auch als Reibmartensit bzeichnet.

### 6. Beispiel (Variante I):

Dieses Beispiel betrifft eine Endoprothese aus einem Mehrlagenverbundwerkstoff.

Zuerst wird ein Fe-Rohr mit einer Wanddicke von 40 µm und einem Kohlenstoffgehalt von 0,1 bis 0,2 Gew.% aus einem C15-Stahl oder einem C10-Stahl, wobei der letztgenannte 0,07 Gew.% bis 0,13 Gew.% C (die anderen Elemente wie beim oben beschriebenen C15-Stahl) enthält, in der Matrix gezogen. Danach erfolgt eine erste Wärmebehandlung in einer kohlenstoffhaltigen Atmosphäre bei ca. 900°C mit einer Aufkohlungstiefe von 5 bis 20 µm.

Anschließend wird ein zweites Rohr gezogen und über dem bereits aufgekohlten ersten Rohr angeordnet, so dass es einen Mantel um das erste Rohr bildet. Das zweite Rohr hat ebenfalls eine Wandstärke von etwa 40 µm, so dass die gesamte Wandstärke des Stentkörpers nun 80 µm beträgt. Bei Verwendung von drei Rohren würde die Gesamt-Wandstärke des Stentkörpers dementsprechend 120 µm betragen. Anschließend wird eine zweite thermochemische Behandlung in einer kohlenstoffhaltigen Atmosphäre bei 900°C durchgeführt und eine Aufkohlung bis in eine Tiefe von 5 bis 20 µm erzeugt. Hierbei wird einerseits die äußere Oberfläche des Rohrs aufgekohlt, andererseits diffundiert der Kohlenstoff aus der mittels des ersten Aufkohlungsschritts erzeugten Grenzschicht des ersten Rohrs aus dem Interface in das zweite Rohr und bildet somit eine Verbindungszone (Verbindungsschicht) zwischen erstem Rohr und zweitem Rohr (bzw. zwischen dem zweiten und dem dritten Rohr). Diese Verbindungszone erhöht die Adhäsion zwischen den jeweiligen Rohren und minimiert auch den Kohlenstoff-Gradienten im Übergang zwischen zwei Rohren. Die letzten beiden Verfahrensschritte können so lange wiederholt werden, bis der Stent die gewünschte Wandstärke erreicht hat.

Zusätzlich kann die Oberfläche des Stentkörpers nach Abschluss der Wärmebehandlungen mit chloridhaltigen Medien tribochemisch behandelt werden. Eine solche tribochemische Behandlung kann auch im Anschluss an die anhand der Beispiele 1 bis 5 hergestellten Implantate durchgeführt werden.

Die für die tribochemische Behandlung verwendeten Salze bestehen aus einer Mischung von jeweils 50 Masse% NaCl und MgCl₂. Die Strahlteilchen weisen eine Teilchengröße auf, die zwischen 200 nm und 20 µm variiert. Mittels Druckluft bei 3 bis 4 bar Druck werden die Strahlteilchen mittels einer Strahlanlage gleichzeitig auf die Innen- und die Außenseite des Implantates gestrahlt. Der Prozess erfolgt über einen Zeitraum von 5 min. Um ein Verklumpen der hygroskopischen Salzmischung zu vermeiden, muss diese vor und während des tribochemischen Behandlungsprozesses trocken gelagert werden.

Die Chlorid-Strahlteilchen werden beim Auftreffen auf die Oberfläche des Implantats in viele kleine Fragmente zerlegt, welche teilweise von der Oberfläche zurückreflektiert werden und an dieser abprallen. Die Fragmente der Strahlteilchen, welche an der Oberfläche adhärieren, reagieren in der oben beschriebenen Weise mit dem Implantatmaterial und erzeugen die Submikroaufrauungen. Ggf. ebenfalls verwendete Hartstoffpartikel erzeugen Mikroaufrauungen.

In Figur 1 ist schematisch ein Querschnitt eines nach dem in Beispiel 6 angegebenen Verfahren hergestellten Stentkörpers gezeigt. Der Stentkörper setzt sich aus zwei übereinander angeordneten Rohren zusammen, nämlich einem ersten inneren Rohr 1 und einem zweiten äußeren Rohr 2. Das erste Rohr 1 besteht aus C 15-Stahl und das zweite Rohr 2 aus einem C10-, C15 oder C20-Stahl. In dem Grenzbereich zwischen dem ersten Rohr 1 und dem zweiten Rohr 2 ist eine aufgekohlte Verbindungszone 5 angeordnet. Ferner ist eine aufgekohlte Randzone 6 in einem außen liegenden Grenzbereich des zweiten Rohrs 2 vorgesehen. Durch die oben angegebene tribochemische Behandlung ist ferner eine gegenüber Bereich 6 weiter außen liegende Grenzzone 7 angeordnet, in der das Material mittels Chloridionen tribochemisch behandelt wurde. Wenn die tribochemische Behandlung nicht durchgeführt wurde, entfällt diese Zone 7. Die in Figur 1 gezeigten Schichtdicken sind nicht maßstäblich.

## Patentansprüche

1. Implantat, insbesondere intraluminale Endoprothese, mit einem Körper enthaltend eine Eisenbasislegierung , wobei in dem Gefüge der oberflächennahen Grenzschicht mindestens eines Teils des Implantatkörpers gegenüber der jeweiligen Konzentration im restlichen Gefüge
- durch Aufkohlen ein erhöhter Kohlenstoffanteil in einer Konzentration von 0,5 Gew.% bis 2 Gew.%, oder
- durch Borieren ein erhöhter Boranteil in einer Konzentration von 1 Gew.% bis 20 Gew.%, oder
- durch Nitrocarburieren ein erhöhter Kohlenstoff- und Stickstoffanteil in einer Konzentration von Kohlenstoff von 0,5 Gew.% bis 7 Gew.%, und von Stickstoff von 0,5 Gew.% bis 17 Gew.%, oder
- durch Entkohlen ein verringerter Kohlenstoffanteil in einer Konzentration von 0,1 Gew.% bis 0,3 Gew.%,
vorliegt.

2. Implantat nach Anspruch 1, wobei in dem Gefüge der oberflächennahen Grenzschicht mindestens eines Teils des Implantatkörpers gegenüber der jeweiligen Konzentration im restlichen Gefüge durch Aufkohlen ein erhöhter Kohlenstoffanteil in einer Konzentration von 0,8 Gew.% bis 1,3 Gew.% vorliegt.

3. Implantat nach Anspruch 1 oder 2, wobei in dem Gefüge der oberflächennahen Grenzschicht mindestens eines Teils des Implantatkörpers gegenüber der jeweiligen Konzentration im restlichen Gefüge durch Borieren ein erhöhter Boranteil in einer Konzentration von 7 Gew.% bis 10 Gew.% vorliegt.

4. Implantat nach einem der Ansprüche 1 bis 3, wobei in dem Gefüge der oberflächennahen Grenzschicht mindestens eines Teils des Implantatkörpers gegenüber der jeweiligen Konzentration im restlichen Gefüge durch Nitrocarburieren ein erhöhter Kohlenstoff- und Stickstoffanteil in einer Konzentration von Kohlenstoff von 0,5 Gew.% bis 4 Gew.%, bevorzugt von 0,5 Gew.% bis 2 Gew.%, und von Stickstoff von 5 Gew.% bis 12 Gew.% vorliegt.

5. Implantat nach einem der Ansprüche 1 bis 4, wobei in dem Gefüge der oberflächennahen Grenzschicht mindestens eines Teils des Implantatkörpers gegenüber der jeweiligen Konzentration im restlichen Gefüge durch Entkohlen ein verringerter Kohlenstoffanteil in einer Konzentration von 0,15 Gew.% bis 0,25 Gew.% vorliegt.

6. Implantat nach einem der Ansprüche 1 bis 5, **dadurch gekennzeichnet, dass** das Implantat in und/oder nahe der Oberfläche Poren aufweist und/oder dass die Oberfläche des Implantatkörpers zumindest teilweise tribochemisch aufgebrachte Strahlteilchen und/oder eine Beschichtung aufweist, welche eine pharmazeutisch aktive Substanz enthält.

7. Implantat nach einem der Ansprüche 1 bis 6, **dadurch gekennzeichnet, dass** der Körper des Implantats überwiegend Eisen aufweist, vorzugsweise mehr als 80 Gew.%, besonders bevorzugt mehr als 99 Gew.% Eisen, insbesondere in einer Legierung, enthält.

8. Implantat nach einem der Ansprüche 1 bis 7, **dadurch gekennzeichnet, dass** der Körper des Implantats in der durch die Wärmebehandlung entstandenen Grenzschicht einen Anteil von maximal 40 Gew.% Zementit aufweist.

## Claims

1. An implant, in particular an intraluminal endoprosthesis, comprising a body containing an iron-based alloy, wherein, in the structure of the near-surface boundary layer of at least part of the implant body, compared with the respective concentration in the rest of the structure,
- an increased carbon proportion in a concentration from 0.5% by weight to 2% by weight by carburising, or
- an increased boron proportion in a concentration from 1% by weight to 20% by weight by boronising, or
- an increased carbon and nitrogen proportion in a concentration of carbon from 0.5% by weight to 7% by weight and of nitrogen from 0.5% by weight to 17% by weight by nitrocarburising, or
- a reduced carbon proportion in a concentration from 0.1% by weight to 0.3% by weight by decarburising
is present.

2. The implant according to Claim 1, wherein, in the structure of the surface-near boundary layer of at least part of the implant body, compared with the respective concentration in the rest of the structure, an increased carbon proportion in a concentration from 0.8% by weight to 1.3% by weight is present by carburising.

3. The implant according to Claim 1 or 2, wherein, in the structure of the surface-near boundary layer of at least part of the implant body, compared with the respective concentration in the rest of the structure, an increased boron proportion in a concentration from 7% by weight to 10% by weight is present by boronising.

4. The implant according to one of Claims 1 to 3, wherein, in the structure of the surface-near boundary layer of at least part of the implant body, compared with the respective concentration in the rest of the structure, an increased carbon and nitrogen proportion in a concentration of carbon from 0.5% by weight to 4% by weight, preferably from 0.5% by weight to 2% by weight, and of nitrogen from 5% by weight to 12% by weight is present by nitrocarburising.

5. The implant according to one of Claims 1 to 4, wherein, in the structure of the surface-near boundary layer of at least part of the implant body, compared with the respective concentration in the rest of the structure, a reduced carbon proportion in a concentration from 0.15% to 0.25% by weight is present by decarburising.

6. The implant according to one of Claims 1 to 5, **characterised in that** the implant has pores in and/or close to the surface, and/or **in that** the surface of the implant body comprises at least partially tribochemically applied beam particles and/or a coating which contains a pharmaceutically active substance.

7. The implant according to one of Claims 1 to 6, **characterised in that** the body of the implant predominantly contains iron, preferably more than 80% by weight, particularly preferably more than 99% by weight of iron, particularly in an alloy.

8. The implant according to one of Claims 1 to 7, **characterised in that** the body of the implant in the boundary layer produced by the heat treatment contains a proportion of at most 40% by weight of cementite.

## Revendications

1. Implant, en particulier une endoprothèse, avec un corps contenant un alliage à base de fer, dans lequel on trouve, dans la texture de la couche limite proche de la surface d'au moins une partie du corps d'implant,
- une part de carbone augmentée par carburation, selon une concentration de 0,5% en poids à 2% en poids, ou
- une part de bore augmentée par boruration selon une concentration de 1% en poids à 20% en poids, ou
- une part de carbone et d'azote augmentée par nitrocarburation selon une concentration de carbone de 0,5% en poids à 7% en poids, et une concentration d'azote de 0,5% en poids à 17% en poids, ou
- une part de carbone diminuée par décarburation, selon une concentration de 0,1 % en poids à 0,3% en poids,
par rapport à la concentration respective dans le reste de la structure.

2. Implant selon la revendication 1, dans lequel on trouve, dans la structure de la couche limite proche de la surface d'au moins une partie du corps d'implant, une part de carbone augmentée par carburation selon une concentration de 0,8% en poids à 1,3% en poids, par rapport à la concentration respective du reste de la structure.

3. Implant selon la revendication 1 ou 2, dans lequel on trouve, dans la structure de la couche limite proche de la surface d'au moins une partie du corps d'implant, une part de bore augmentée par boruration selon une concentration de 7% en poids à 10% en poids, par rapport à la concentration respective du reste de la structure.

4. Implant selon l'une des revendications 1 à 3, dans lequel on trouve, dans la structure de la couche limite proche de la surface d'au moins une partie du corps d'implant, une part de carbone et d'azote augmentée par nitrocarburation, selon une concentration de carbone de 0,5% en poids à 4% en poids, de préférence de 0,5% en poids à 2% en poids, et une concentration d'azote de 5% en poids à 12% en poids, par rapport à la concentration respective du reste de la structure.

5. Implant selon l'une des revendications 1 à 4, dans lequel on trouve, dans la structure de la couche limite proche de la surface d'au moins une partie du corps d'implant, une part de carbone diminuée par décarburation selon une concentration de 0,15% en poids à 1,25% en poids, par rapport à la concentration respective du reste de la structure.

6. Implant selon l'une des revendications 1 à 5, **caractérisé en ce que** l'implant comporte des pores dans et/ou à proximité de la surface, et/ou **en ce que** la surface du corps d'implant comporte des particules abrasives appliquées au moins partiellement de façon tribochimique et/ou un revêtement contenant une substance pharmaceutique active.

7. Implant selon l'une des revendications 1 à 6, **caractérisé en ce que** le corps de l'implant contient majoritairement du fer, de préférence plus de 80% en poids, de façon particulièrement préférée plus de 99% en poids de fer, en particulier un alliage.

8. Implant selon l'une des revendications 1 à 7, **caractérisé en ce que** le corps de l'implant comporte une part maximale de 40% en poids de cémentite dans la couche limite produite par traitement thermique.
